# EUROPEAN PATENT SPECIFICATION

(11) **EP 2 773 747 B1**
(45) Date of publication and mention of the grant of the patent: **09.01.2019**
(21) Application number: 12844896.6
(22) Date of filing: 01.11.2012
(51) Int. Cl.: A61P 37/06, A61K 38/16, A61K 35/28

(54) **TSG-6 PROTEIN FOR USE IN PREVENTION AND TREATMENT OF REJECTION OF A CORNEAL TRANSPLANT**
TSG-6 PROTEIN ZUR VERWENDUNG BEI DER VORBEUGUNG UND BEHANDLUNG DER ABSTOSSUNG EINER HORNHAUTTRANSPLANTATION
LA ROTÉINE TSG-6 POUR LE PRÉVENTION OU LE TRAITEMENT DU REJET D'UNE GREFFE DE CORNÉE

(30) Priority: 04.11.2011 US 201161555717 P
(43) Date of publication of application: 10.09.2014
(73) Proprietor: The Texas A & M University System, College Station, TX 77845 (US); Scott & White Healthcare, Temple, TX 76508 (US)
(72) Inventor: YU, Jimin, College Station, TX 77845 (US); PROCKOP, Darwin J., Philadelphia, PA 19106 (US); OH, Joo Youn, Seoul 110-1744 (KR); LEE, Ryanghwa, Temple, TX 76502 (US)
(74) Representative: Louis Pöhlau Lohrentz
(86) International application number: PCT/US2012/062972
(87) International publication number: WO 2013/067124

(56) References cited:
- WO-A1-2011/060244
- US-B1- 6 368 636
- HELGA REINSHAGEN ET AL: "Corneal surface reconstruction using adult mesenchymal stem cells in experimental limbal stem cell deficiency in rabbits", ACTA OPHTHALMOLOGICA, vol. 89, no. 8, 21 December 2009 (2009-12-21), pages 741-748, XP055167417, ISSN: 1755-375X, DOI: 10.1111/j.1755-3768.2009.01812.x
- ON BEHALF OF THE DEVELOPMENTAL COMMITTEE OF THE EUROPEAN GROUP FOR ET AL: "Mesenchymal stem cells for treatment of steroid-resistant, severe, acute graft-versus-host disease: a phase II study", THE LANCET, THE LANCET PUBLISHING GROUP, GB, vol. 371, no. 9624, 10 May 2008 (2008-05-10), pages 1579-1586, XP026859188, ISSN: 0140-6736, DOI: 10.1016/S0140-6736(08)60690-X [retrieved on 2008-05-10]
- ALMA J NAUTA AND WILLEM E FIBBE: "Immunomodulatory properties of mesenchymal stromal cells", BLOOD, AMERICAN SOCIETY OF HEMATOLOGY, US, vol. 110, no. 10, 1 November 2007 (2007-11-01), pages 3499-3506, XP007913721, ISSN: 0006-4971, DOI: 10.1182/BLOOD-2007-02-069716 [retrieved on 2007-07-30]
- JIANG WANG ET AL: "Control of allograft rejection in mice by applying a novel neuropeptide, cortistatin", ADVANCES IN THERAPY, vol. 25, no. 12, 25 December 2008 (2008-12-25), pages 1331-1341, XP055193405, ISSN: 0741-238X, DOI: 10.1007/s12325-008-0121-z
- YOSHIHISA OKAMOTO ET AL: "Adiponectin Inhibits Allograft Rejection in Murine Cardiac Transplantation", TRANSPLANTATION, vol. 88, no. 7, 1 October 2009 (2009-10-01), pages 879-883, XP055193415, ISSN: 0041-1337, DOI: 10.1097/TP.0b013e3181b6efbf
- N. A. TURGEON ET AL: "Experience with a Novel Efalizumab-Based Immunosuppressive Regimen to Facilitate Single Donor Islet Cell Transplantation", AMERICAN JOURNAL OF TRANSPLANTATION, vol. 10, no. 9, 25 August 2010 (2010-08-25), pages 2082-2091, XP055193433, ISSN: 1600-6135, DOI: 10.1111/j.1600-6143.2010.03212.x
- SENSEBE ET AL.: 'Mesenchymal stem cells for clinical application.' VOX SANG vol. 98, no. 2, February 2010, pages 93 - 107, XP055068916
- OH ET AL.: 'Anti-inflammatory protein TSG-6 reduces inflammatory damage to the cornea following chemical and mechanical injury.' PROC NAT ACAD SCI vol. 107, no. 39, 28 September 2010, pages 16875 - 16880, XP055068917
- RODDY ET AL.: 'Action at a distance: systemically administered adult stem/progenitor cells (MSCs) reduce inflammatory damage to the cornea without engraftment and primarily by secretion of TNF-alpha stimulated genelprotein 6.' STEM CELLS vol. 29, no. 10, October 2011, pages 1572 - 1579, XP055068919

## Description

Transplants of organs provide an important therapy for a variety of devastating diseases. However, the therapies are limited by rejection of the transplants by the immune system. Moreover, prolonged administration of immunosuppressive agents to prevent rejection can produce renal or hepatic toxicity and increase the susceptibility to malignancies or infections. One recent strategy for improving transplants of cells and organs is the administration of mesenchymal stem/progenitor cells (MSCs). Systemic infusion of MSCs was reported to decrease the graft rejection in animal models, and the results have prompted a number of clinical trials in patients.¹⁻⁴ Previous studies largely attributed the improved survival of transplants to the immunomodulatory effects of MSCs.³⁻⁶ Most of the data, however, were based on the *in vitro* experiments that may or may not reflect actions of the MSCs *in vivo.*

In order to define how MSCs might improve the engraftment of organ transplants, we here adopted a mouse model of allogeneic corneal transplantation, a model in which the temporal sequence of events from the introduction of the alloantigen to immune rejection is distinct and well-established.⁷⁻⁹ We demonstrated that intravenous (IV) administration of human MSCs (hMSCs) at the time of grafting decreased the immune rejection and prolonged the survival of corneal allograft primarily by suppressing the surgery-induced inflammation in the early postoperative period. Suppression of inflammation subsequently inhibited the afferent loop of the alloimmune response. Of special interest was that the beneficial effects of the MSCs were observed without the cells being engrafted in the cornea, but they were dependent on MSCs trapped in the lungs after IV infusion being activated to express the gene for the anti-inflammatory protein TSG-6.

In accordance with an aspect of the present disclosure, there is provided a method of preventing or treating rejection of a corneal transplant in an animal. The method comprises administering to an animal a composition comprising mesenchymal stem cells, or MSCs. The mesenchymal stem cells are present in the composition in an amount effective to prevent or treat rejection of a corneal transplant in an animal.

In a non-limiting aspect, the mesenchymal stem cells are administered to the animal prior to the corneal transplant. In another non-limiting aspect, the mesenchymal stem cells are administered to the animal concurrently with the corneal transplant. In yet another non-limiting aspect, the mesenchymal stem cells are administered to the animal subsequent to the corneal transplant.

In a further non-limiting aspect, the mesenchymal stem cells are administered to the animal prior to and concurrently with the corneal transplant. In another non-limiting aspect, the mesenchymal stem cells are administered to the animal prior to and subsequent to the corneal transplant. In another non-limiting aspect, the mesenchymal stem cells are administered to the animal concurrently with and subsequent to the corneal transplant. In yet another non-limiting aspect, the mesenchymal stem cells are administered to the animal prior to, concurrently with, and subsequent to the corneal transplant.

In another non-limiting aspect, the mesenchymal stem cells are administered to the animal one day before and immediately after the corneal transplant.

The mesenchymal stem cells may be administered to any animal having an eye with a cornea. Such animals include mammals, including human and non-human primates, birds, reptiles, amphibians and fish.

The MSCs can be obtained from any source. The MSCs may be autologous with respect to the recipient (obtained from the same host) or allogeneic with respect to the recipient. In addition, the MSCs may be xenogeneic to the recipient (obtained from an animal of a different species), for example rat MSCs may be used to treat or prevent transplant rejection, including corneal transplant rejection, in a human.

In a further non-limiting aspect, MSCs used in the present invention can be isolated, from the bone marrow of any species of mammal, including but not limited to, human, mouse, rat, ape, gibbon, bovine. In a non-limiting aspect, the MSCs are isolated from a human, a mouse, or a rat. In another non-limiting aspect, the MSCs are isolated from a human.

Any medium capable of supporting MSCs in vitro may be used to culture the MSCs. Media formulations that can support the growth of MSCs include, but are not limited to, Dulbecco's Modified Eagle's Medium (DMEM), alpha modified Minimal Essential Medium (αMEM), and Roswell Park Memorial Institute Media 1640 (RPMI Media 1640) and the like. Typically, 0 to 20% fetal bovine serum (FBS) or 1-20% horse serum is added to the above medium in order to support the growth of MSCs. A defined medium, however, also can be used if the growth factors, cytokines, and hormones necessary for culturing MSCs are provided at appropriate concentrations in the medium. Media useful in the methods may contain one or more compounds of interest, including but not limited to antibiotics, mitogenic or differentiation compounds useful for the culturing of MSCs. The cells may be grown in one non-limiting embodiment, at temperatures between 27°C to 40°C. in another non-limiting embodiment at 31°C to 37°C, and in another non-limiting embodiment in a humidified incubator. The carbon dioxide content may be maintained between 2% to 10% and the oxygen content may be maintained between 1% and 22%; however, the invention should in no way be construed to be limited to any one method of isolating and culturing MSCs. Rather, any method of isolating and culturing MSCs should be construed to be included in the present disclosure.

Antibiotics which can be added into the medium include, but are not limited to, penicillin and streptomycin. The concentration of penicillin in the culture medium is about 10 to about 200 units per ml. The concentration of streptomycin in the culture medium is about 10 to about 200 µg/ml.

Once the mesenchymal stem cells are obtained from a source as hereinabove described, and are cultured to provide a sufficient number of mesenchymal stem cells, the mesenchymal stem cells are administered to the animal to treat rejection of the corneal transplant.

The mesenchymal stem cells are administered by any of a variety of acceptable means of administration known to those of ordinary skill in the art. Such methods include, but are not limited to, intravenous, intraperitoneal, intramuscular, intradermal, subcutaneous or topical administration.

Applicants have discovered that, when mesenchymal stem cells are administered to an animal to prevent or treat rejection of a corneal transplant, the mesenchymal stem cells are activated in vivo to increase significantly expression of the anti-inflammatory protein known as tumor necrosis factor stimulated gene 6 protein, or TSG-6 protein, which reduces inflammation induced as a result of the corneal transplant. It is believed further that, by reducing inflammation induced as a result of the corneal transplant, the engraftment of the transplanted cornea is facilitated and improved.

As noted hereinabove, it is believed that TSG-6 protein reduces inflammation induced by a corneal transplant, thereby improving the engraftment of the transplanted cornea. It is believed further that anti-inflammatory proteins, such as TSG-6, may improve the engraftment of and/or treat or prevent the rejection of other cells, tissues, or organs, as well as aid in the survival of implants of prostheses or alleviate inflammation induced by surgery. The present invention is defined by the claims.

Thus, in accordance with an embodiment of the present invention, there is provided a composition comprising TSG-6 protein or biologically active fragment thereof for use in a method of preventing and/or treating rejection of a corneal transplant in an animal.

Such biologically active fragments of TSG-6 protein include, but are not limited to, the TSG-6 link module (i.e., amino acid residues 1 through 133 of the "native" human TSG-6 protein), and TSG-6 protein or biologically active fragments thereof having at least one histidine residue at the C-terminal thereof.

Native human TSG-6 protein has the following amino acid sequence:

The TSG-6 link module consists of amino acid residues 1 through 133 of the above sequence.

In a non-limiting embodiment, TSG-6 protein or biologically active fragment thereof, is administered prior to the transplant of the organ. In another non-limiting embodiment, TSG-6 protein or biologically active fragment thereof is administered concurrently with the transplanted organ. In yet another non-limiting embodiment, the TSG-6 protein or biologically active fragment thereof is administered subsequent to the transplant of the organ.

In another non-limiting embodiment, the TSG-6 protein or biologically active fragment thereof is administered prior to and concurrently with the transplant of the organ. In another non-limiting embodiment, the TSG-6 protein or biologically active fragment thereof is administered prior to and subsequent to the transplant of the organ. In yet another non-limiting embodiment, the TSG-6 protein or fragment thereof is administered prior to, concurrently with, and subsequent to the transplant of the organ.

The TSG-6 protein or biologically active fragment thereof may be administered to any animal that is a recipient of a transplant of the organ. Such animals include those hereinabove described, including mammals, and including human and non-human primates.

In another non-limiting embodiment, the TSG-6 protein or biologically active fragment thereof is administered in combination with mesenchymal stem cells.

The TSG-6 protein or biologically active fragment thereof is administered by any acceptable means known to those skilled in the art, such as intravenous, peritoneal, intramuscular, intradermal, subcutaneous, or topical administration.

In another non-limiting embodiment, the TSG-6 protein or biologically active fragment thereof may be administered in combination with at least one immunoregulator and/or at least one immunosuppressive agent.

The TSG-6 protein or a biologically active fragment thereof is administered in conjunction with an acceptable pharmaceutical carrier.

Suitable excipients for injection solutions are those that are biologically and physiologically compatible with the MSCs and/or TSG-6 protein and with the recipient, such as buffered saline solution or other suitable excipients. The composition for administration can be formulated, produced and stored according to standard methods complying with proper sterility and stability.

The dosage of the MSCs and/or TSG-6 protein varies within wide limits and may be adjusted to the individual requirements in each particular case. The number of cells and/or amount of TSG-6 protein used depends on the weight and condition of the recipient, the number and/or frequency of administrations, and other variables known to those of skill in the art, including, but not limited to, the age and sex of the patient, and the extent and severity thereof.

TSG-6 protein can be obtained by any method. For example, in a non-limiting embodiment, the protein can be purified from a transgenic cell that (a) comprises a heterologous nucleotide sequence encoding the protein, and (b) expresses the protein.

Any cell that may be transformed to express a heterologous nucleotide sequence may be used to express TSG-6 protein, or a biologically active fragment thereof Such cells include human and non-human eukaryotic animal cells. In another embodiment, the cell is a non-human eukaryotic animal cell.

In another non-limiting embodiment, the protein may be synthesized by an automatic protein or peptide synthesizer by means known to those skilled in the art.

In a non-limiting embodiment, the TSG-6 protein or a biologically active fragment thereof, may be administered to the eye topically, such as, for example, in the form of eye drops, in order to treat or prevent rejection of a corneal transplant.

Various modifications of the protein, such as addition of polyethylene glycol chains (PEGylation), may be made to influence their pharmacokinetic and/or pharmacodynamic properties.

To administer the protein by other than parenteral administration, the protein may be coated or co-administered with a material to prevent its inactivation. For example, the protein may be administered in an incomplete adjuvant, co-administered with enzyme inhibitors or administered in liposomes. Enzyme inhibitors include pancreatic trypsin inhibitor, disopropylfluorophosphate (DEP) and trasylol. Liposomes include water-in-oil-in-water, CGF emulsions, as well as conventional liposomes (Strejan, et al. (1984) J. Neuroimmunol. 7:27).

Although the compositions of this invention can be administered in simple solution, they are more typically used in combination with other materials such as carriers, preferably pharmaceutical carriers. Useful pharmaceutical carriers can be any compatible, non-toxic substance suitable for delivering the compositions of the invention to a patient. Sterile water, alcohol, fats, waxes, and inert solids may be included in a carrier. Pharmaceutically acceptable adjuvants (buffering agents, dispersing agents) may also be incorporated into the pharmaceutical composition. Generally, compositions useful for parenteral administration of such drugs are well known; e.g., Remington's Pharmaceutical Science, 17th Ed. (Mack Publishing Company, Easton, Pa., 1990). Alternatively, compositions of the invention may be introduced into a patient's body by implantable drug delivery systems [Urquhart et al., Ann. Rev. Pharmacol. Toxicol. 24:199 (1984).

Therapeutic formulations may be administered in many conventional dosage formulations. Formulations typically comprise at least one active ingredient, together with one or more pharmaceutically acceptable carriers.

The formulations conveniently may be presented in unit dosage form and may be prepared by any methods well known in the art of pharmacy. See, e.g., Gilman et al. (eds.) (1990), The Pharmacological Bases of Therapeutics, 8th Ed., Pergamon Press; and Remington's Pharmaceutical Sciences, supra, Easton, Pa.; Avis, et al. (eds.) (1993) Pharmaceutical Dosage Forms: Parenteral Medications Dekker, N.Y.; Lieberman et al. (eds.) (1990), Pharmaceutical Dosage Forms: Tablets, Dekker, N.Y.; and Lieberman et al. (eds.) (1990), Phamiaceutical Dosage Forms: Disperse Systems, Dekker, N.Y.

In another non-limiting embodiment, the mesenchymal stem cells or the protein employed for the claimed treating or preventing rejection of a transplanted organ may be contained in a nanoparticle. Such nanoparticles may be formed by methods known to those skilled in the art, and administered by methods such as those hereinabove described.

The invention now will be described with respect to the drawings. It is to be understood, however, that the scope of the present invention is not intended to be limited thereby.

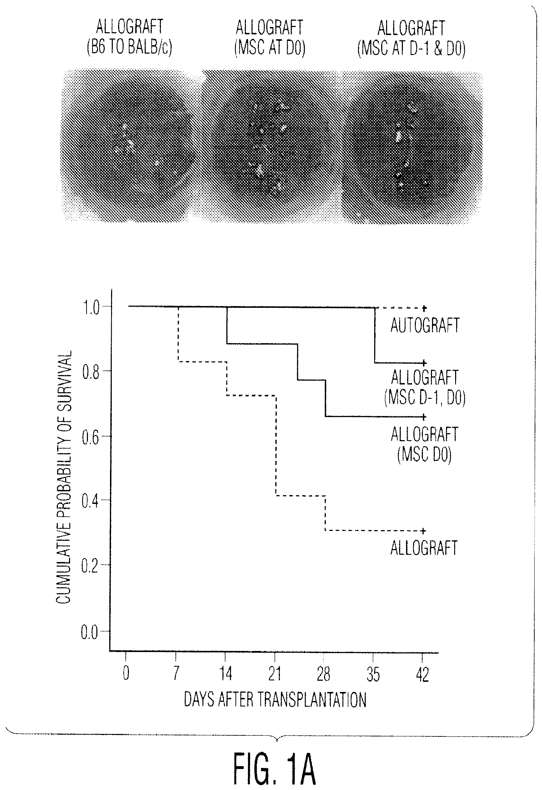

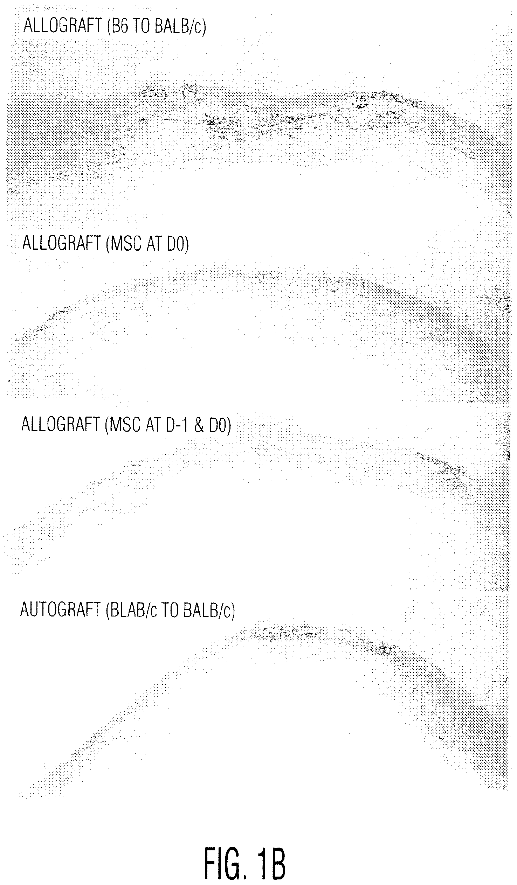

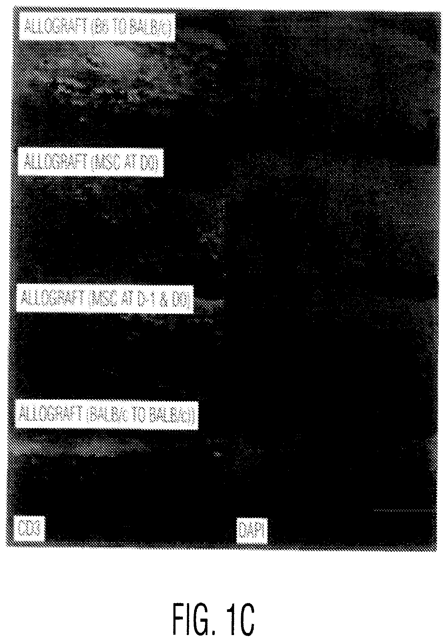

**Figure 1****. IV hMSCs prolonged the survival and prevented the immune rejection of B6 corneal grafts in BALB/c mice. (a)** Representative photographs of the cornea 14 days after transplantation and the Kaplan-Meier survival curve of corneal grafts. The graft survival was prolonged significantly by IV hMSCs. Seven out of 12 B6 corneal grafts in BALB/c mice (allografts) were rejected within 28 days (median survival time 23.1 days). In contrast, 11 out of 12 allografts survived in mice that received IV hMSCs both one day before surgery (D-1) and immediately after surgery (D0), and 9 of 12 allografts survived in mice that received a single injection of hMSCs at DO (MSC at D-1 and 0 vs. HBSS, p=0.006; MSC at D0 vs. HBSS, p=0.047: Generalized Wilcoxon test). n=12 in each group. **(b)** Hematoxylin-eosin staining of corneal grafts at day 28 showed heavy infiltration of inflammatory cells in the rejected allografts of control animals and much less inflammatory cell infiltration in the allografts that received hMSCs. (c) Immunohistochemical staining showed that many CD3⁺ T cells infiltrated the allografts of control animals, whereas there were rare T cells in the grafts that received hMSCs.

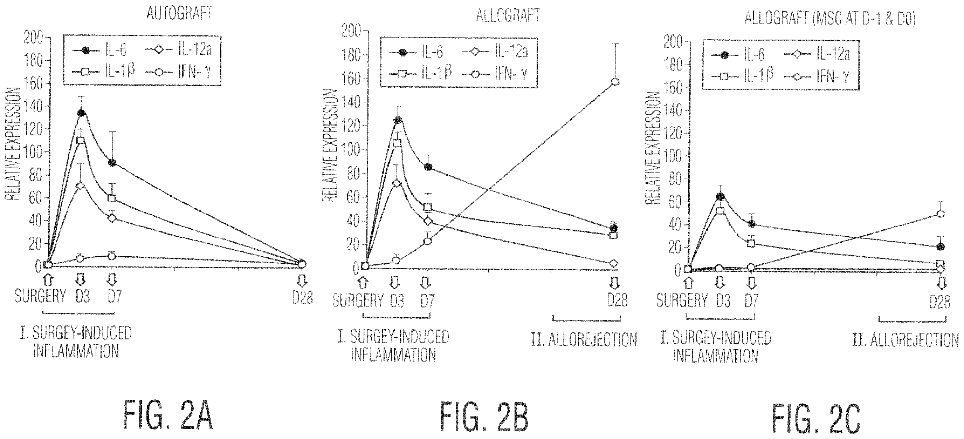

**Figure 2****. Time course of gene expression levels in the cornea after transplantation surgery.** Real-time RT-PCR showed that the levels of pro-inflammatory cytokines (IL-6, IL-1β, and IL-12a) were up-regulated at similar levels in autografts **(a)** and allografts **(b)** at days 3 and 7 after transplantation, which defines the early phase of surgery-induced inflammation. The levels of T cell-derived cytokines (IFN-γ) were increased up to day 28 in allografts, but not in autografts, which indicates the late phase of the allogeneic immune rejection. In allografts that received IV hMSCs (c), levels of IL-6, IL-1β, and IL-12a were significantly lower at days 3 and 7, and levels of IFN-γ were decreased markedly at day 28 compared to autografts or allografts that did not receive hMSCs. n=5 at each time-point in all experimental groups.

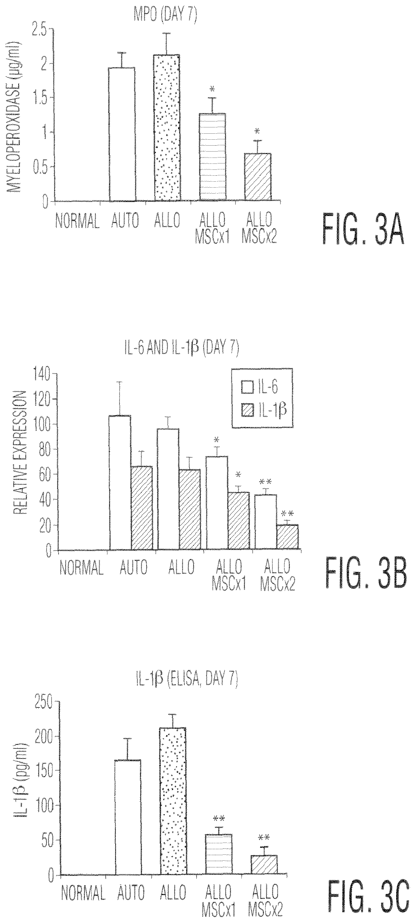

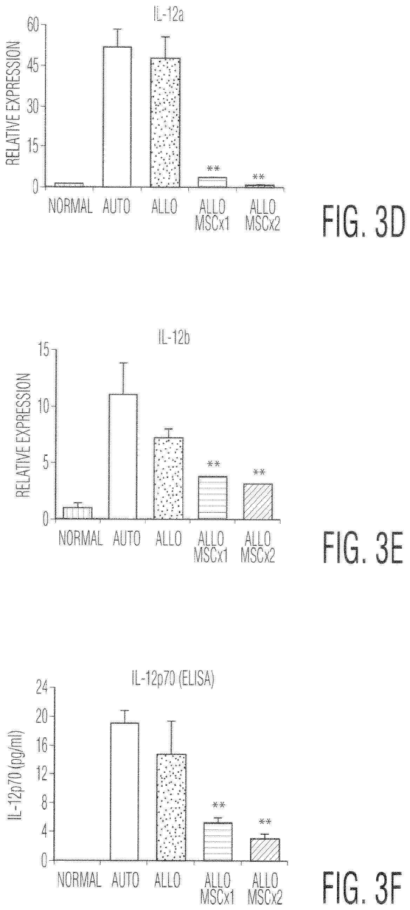

**Figure 3****. IV hMSCs decreased the early inflammatory response in corneal allografts.** The amount of myeloperoxidase as a measure of neutrophil infiltration was decreased significantly by hMSCs at day 7 after transplantation **(a).** Also, the levels of pro-inflammatory cytokines, IL-6, IL-1β, and IL-12 were significantly decreased by IV hMSCs at day 7 **(b-f).** Results indicate that inflammatory responses in the early postoperative period after transplantation surgery were suppressed by IV hMSCs. Auto: autografts, Allo: allografts, Allo MSCx1: allografts that received hMSCs once immediately after transplantation, Allo MSCx2: allografts that received hMSCs on the day before transplantation and immediately after transplantation. n=5 in each group. * *p* < 0.05: ** *p* < 0.01.

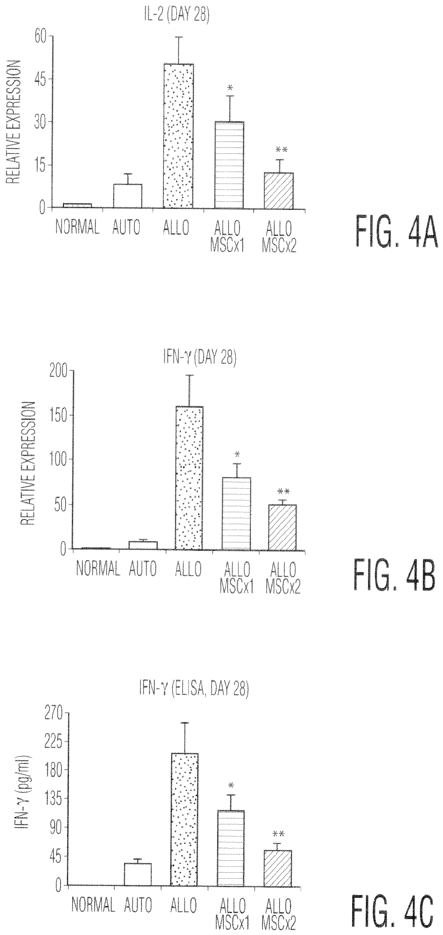

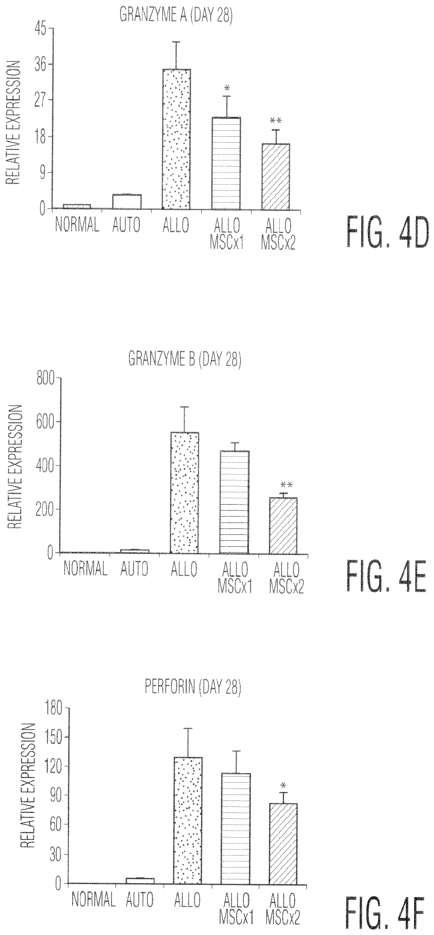

**Figure 4****. IV hMSCs suppressed the late T cell-mediated immune response in corneal allografts.** The transcript levels of activated CD4 T-cell cytokines (IL-2 and IFN-γ) **(a, b)** and protein level of IFN-γ (c) were decreased significantly in the allografts that received IV hMSCs at day 28 after surgery, compared to the grafts without IV hMSCs. Also, the transcript levels of CD8 T-cell effector molecules (granzyme A, granzyme B, and perforin) were decreased significantly by IV hMSCs **(d-f).** Auto: autografts, Allo: allografts, Allo MSCx1: allografts that received hMSCs once immediately after transplantation, Allo MSCx2: allografts that received hMSCs on the day before transplantation and immediately after transplantation. n=5 in each group. **p* < 0.05; ***p <* 0.01.

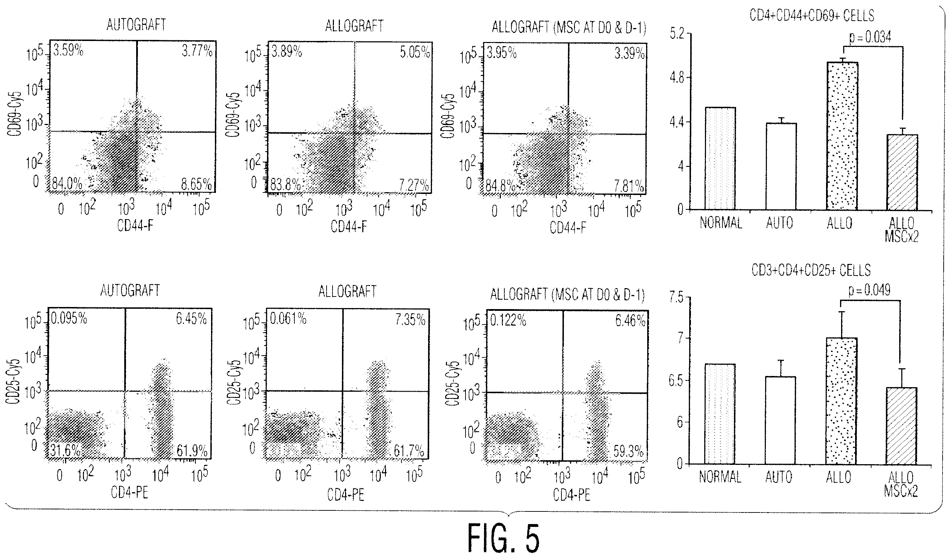

**Figure 5****.** IV hMSCs decreased the number of activated T cells in ipsilateral cervical lymph nodes 4 week following transplantation. Flow cytometry showed that the proportion of CD4+CD44+CD69+ cells in draining lymph nodes as markers for activated T cells was decreased significantly in mice treated with hMSCs.

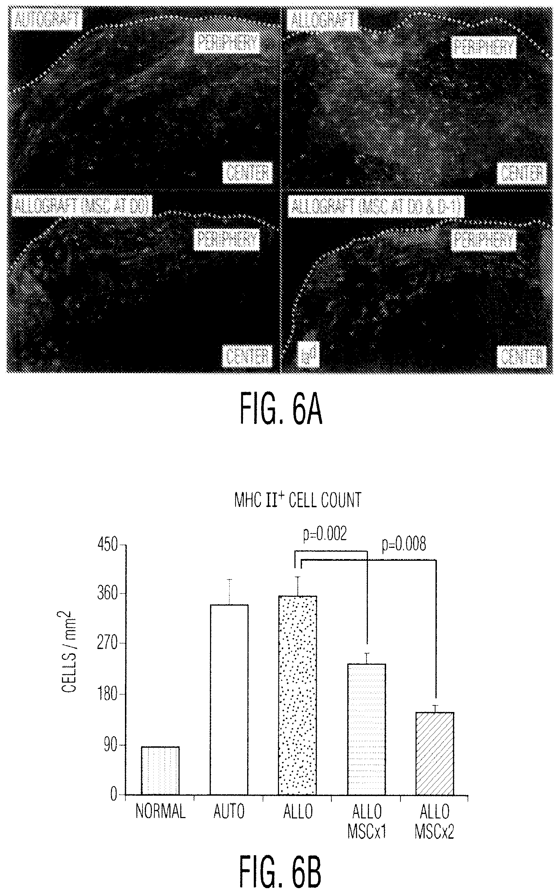

**Figure 6****. IV hMSCs decreased the number of MHC class II⁺ cells in the cornea 1 week following transplantation.** Immunostaining for murine Ia^{d+} **(a)** and enumeration of MHC class II⁺ cells in the whole-mounted epithelial sheets of the cornea **(b)** showed that the number of MHC class II⁺ cells indicating Langerhans cells was increased significantly both in autografts and allografts in response to transplantation surgery; however, the number of MHC class II⁺ cells was decreased significantly in the allografts by IV hMSCs. n=3 in each group. Auto: autografts, Allo: allografts, Allo MSCx1: allografts that received hMSCs once immediately after transplantation, Allo MSCx2: allografts that received hMSCs on the day before transplantation and immediately after transplantation.

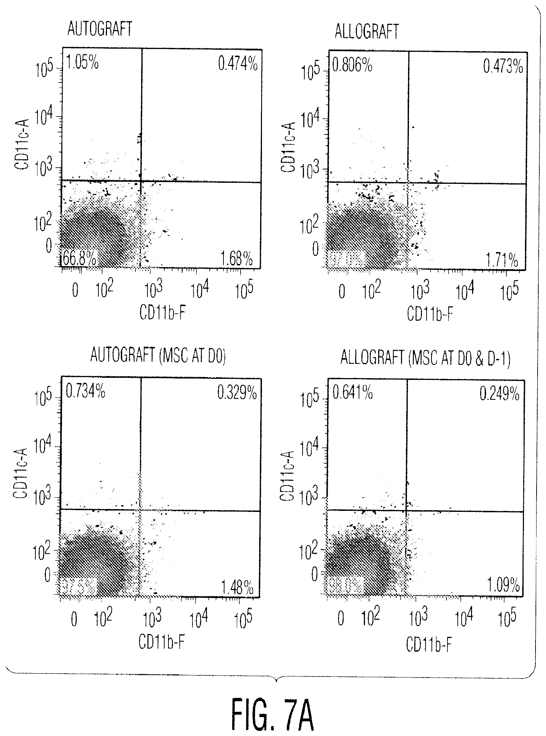

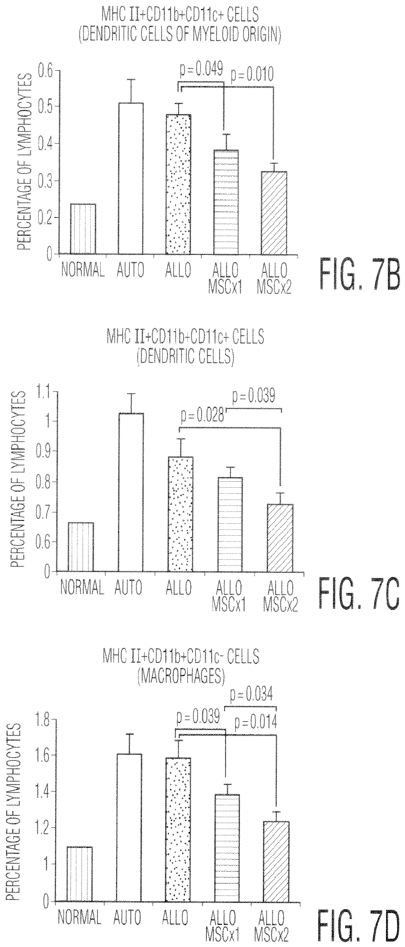

**Figure 7****. IV hMSCs decreased the number of activated antigen presenting cells in ipsilateral cervical lymph nodes 1 week following transplantation. (a)** Flow cytometry showed that the proportions of dendritic cells (DCs), both MHC II⁺ CD11b⁺ CD11c⁺ cells **(b)** and MHC II⁺CD11b⁻ CD11c⁺ cells **(c),** were decreased significantly in mice treated with hMSCs. In addition, the proportion of MHC II⁺ CD11b⁻ CD11c⁺ cells representing macrophages was significantly decreased by IV hMSCs **(d).** n=4 in each group. Auto: autografts, Allo: allografts, Allo MSCx1: allografts that received hMSCs once immediately after transplantation, Allo MSCx2: allografts that received hMSCs on the day before transplantation and immediately after transplantation.

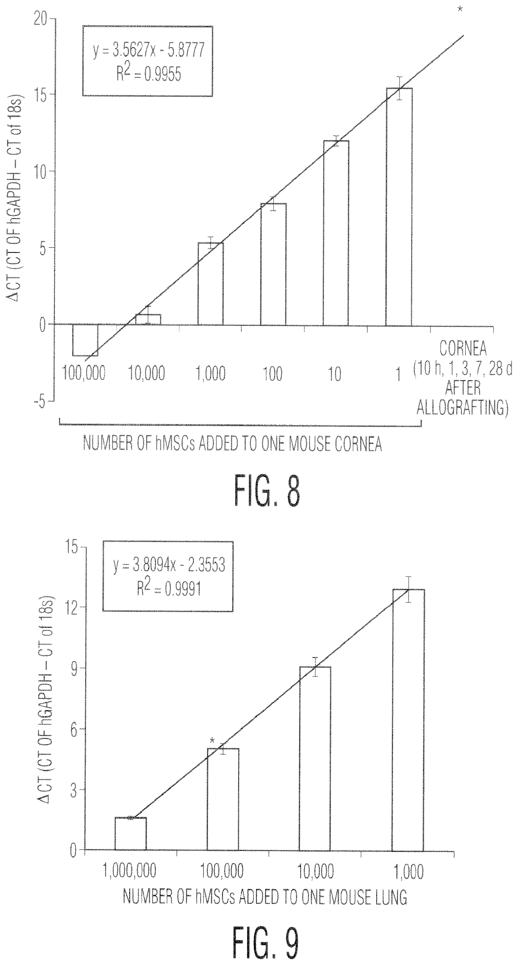

**Figure 8****. The quantitative assay for human mRNA for GAPDH in the cornea after IV administration of hMSCs.** A standard curve of the expression levels of human-specific GAPDH (hGAPDH) in the mouse cornea was constructed by adding known numbers of hMSCs to a single mouse cornea. The expression of hGAPDH was evaluated by real-time RT-PCR to establish a standard curve and to define the detection limits. The assay has the sensitivity to detect 10 hMSCs per one mouse cornea. The level of hGAPDH was assayed in the corneas at 10 hours, 1, 3, 7, and 28 days after 1 x 10⁶ hMSCs were injected IV twice into mice one day before and immediately after corneal allotransplantation surgery. The level of hGAPDH was below the detection limit of the standard curve indicating that less than 10 human cells or <0.001 % of administered cells (1 x 10⁶ cells) reached the cornea.
**Figure 9****. The quantitative assay for human mRNA for GAPDH in the lung after IV administration of hMSCs.**
   A standard curve of the expression levels of human-specific GAPDH (hGAPDH) in the mouse cornea was constructed by adding known numbers of hMSCs to the lung from a single mouse. The expression of hGAPDH was evaluated by real-time RT-PCR to establish a standard curve. The level of hGAPDH was assayed in the lungs at 10 hours after 1 x 10⁶ hMSCs were injected IV twice into mice one day before and immediately after corneal allotransplantation surgery. The asterick indicated the mean level of hGAPDH detected in the lungs from mice treated with hMSCs, demonstrating that about 100,000 human cells (10% of injected cells) were present.

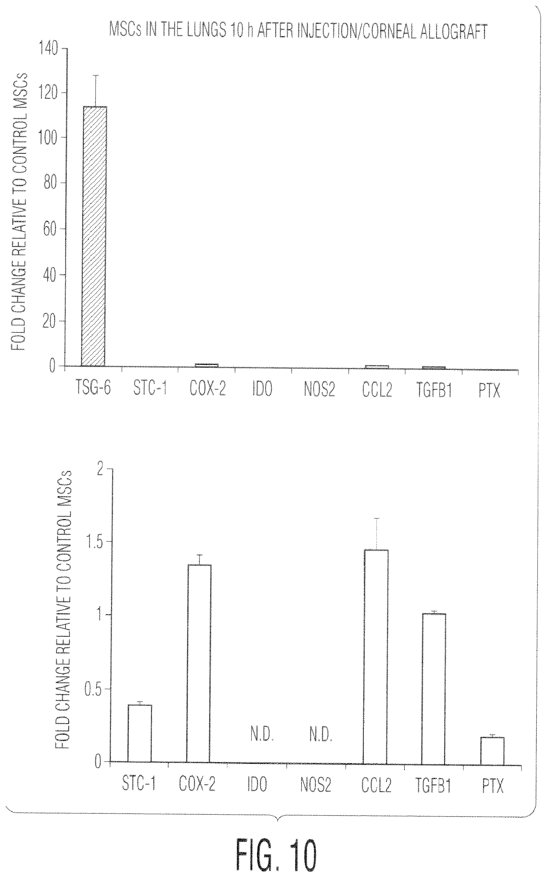

**Figure 10****. Real-time RT-PCR analysis for human-specific transcripts of the anti-inflammatory and immunoregulatory molecules.** RNA was extracted from lungs of the mice 10 hours after infusion of the hMSCs and corneal transplantation surgery and then assayed using real-time RT-PCR for human-specific transcripts of the anti-inflammatory and immunoregulatory molecules, COX2, NOS2, IDO, CCL2, TGF-β, TSG-6, STC-1, and PTX3. Data indicated that the hMSCs trapped in the lungs were activated to up-regulate the expression of the anti-inflammatory molecule TSG-6 up to 113.8-fold, whereas other molecules studied did not show a significant up-regulation.

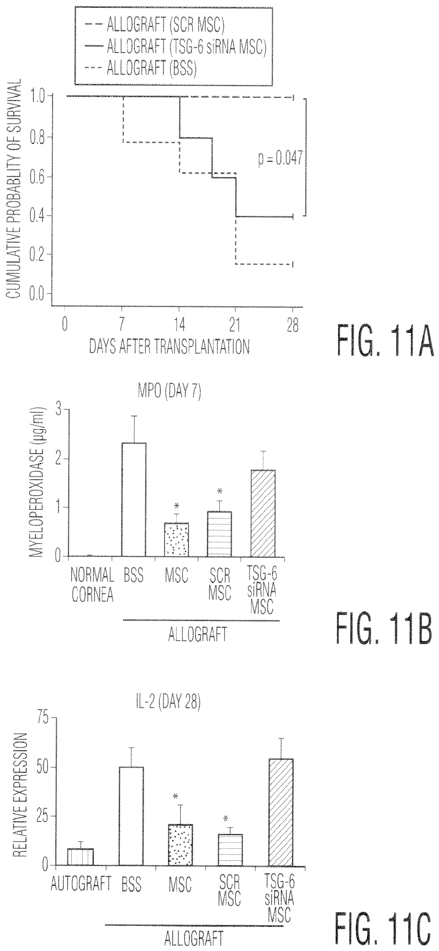

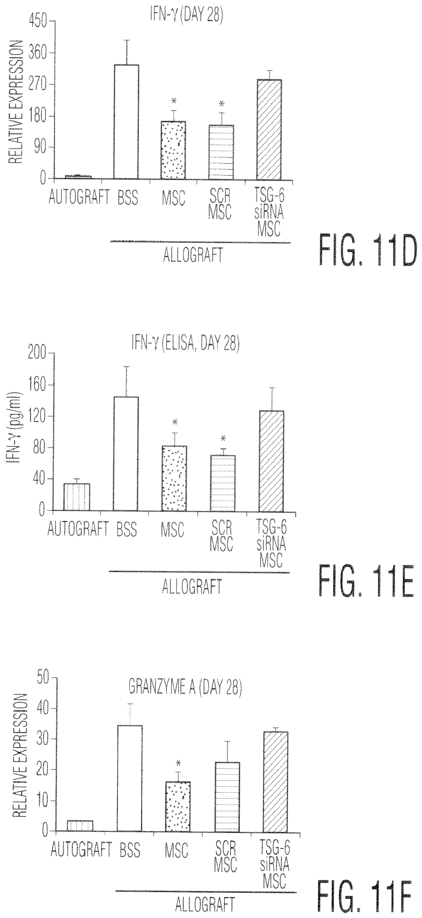

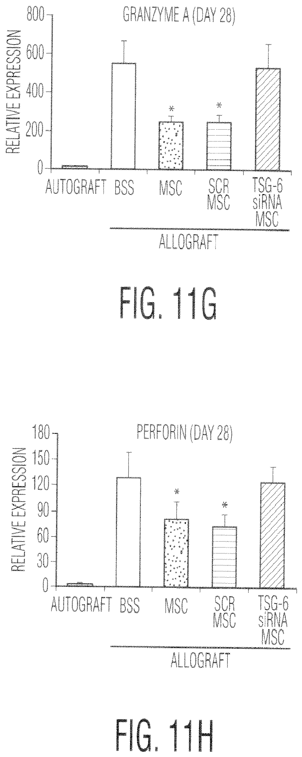

**Figure 11****. IV hMSCs with TSG-6 siRNA knockdown did not suppress the early surgery-induced inflammation and did not prolong the survival of corneal allografts. (a)** The Kaplan-Meier survival curve of B6 corneal grafts in BALB/c mice. Three out of 6 allografts were rejected in mice that received a two-time injection of hMSCs with TSG-6 knockdown (TSG-6 siRNA MSC), whereas all allografts survived in mice that received a two-time injection of hMSCs with scrambled siRNA (SCR MSC). **(b)** The myeloperoxidase (MPO) amount as a measure of neutrophil infiltration was not decreased significantly by hMSCs with TSG-6 knockdown at day 7 after grafting. n=3 in each group. The levels of activated T cell-derived cytokines **(c-e)** and effector enzymes **(f-h)** were not decreased in corneal grafts of mice treated with TSG-6 knockdown hMSCs, whereas hMSCs with scrambled siRNA decreased significantly the levels of T-cell cytokines and enzymes in corneal grafts. n=3 in each group. Auto: autografts, Allo: allografts, Allo MSCx1: allografts that received hMSCs once immediately after transplantation, Allo MSCx2: allografts that received hMSCs on the day before transplantation and immediately after transplantation. n=6 in each group. **p* < 0.05; ***p* < 0.01.

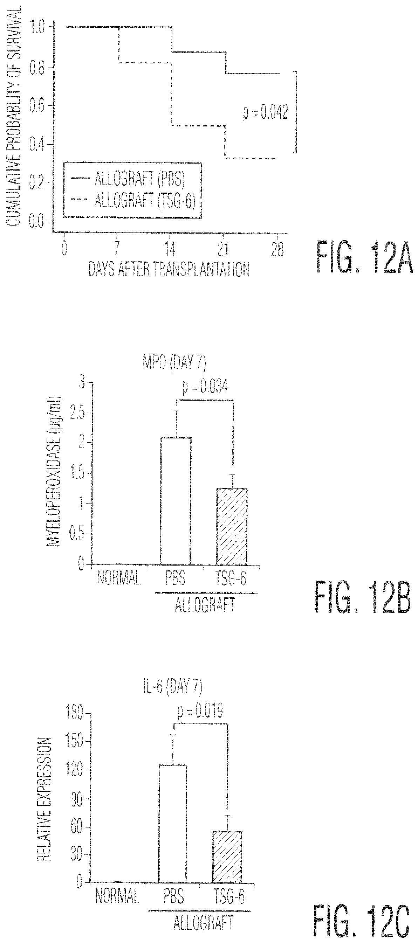

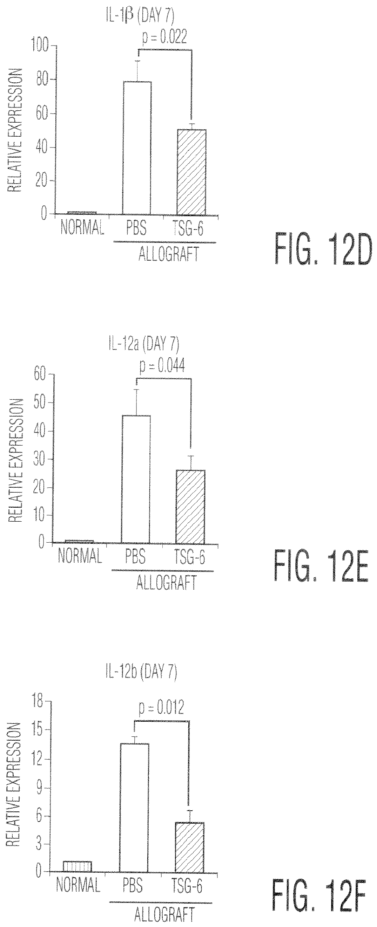

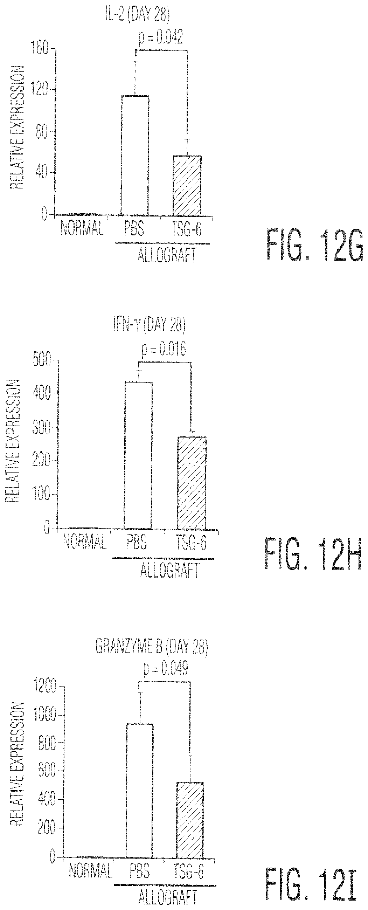

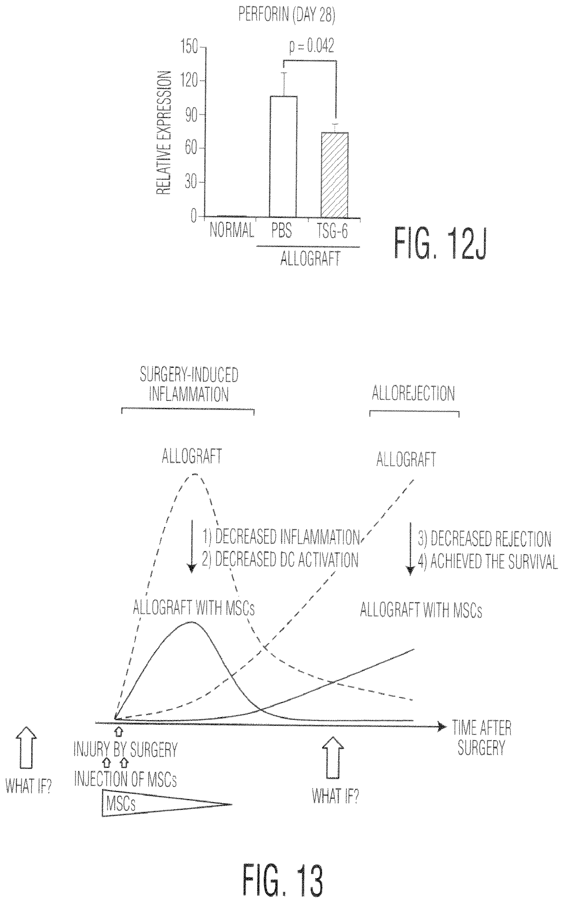

**Figure 12****. IV injection of recombinant TSG-6 suppressed the early surgery-induced** inflammation **and the late immune rejection of the corneal allografts. (a)** The Kaplan-Meier survival curve of B6 corneal grafts in BALB/c mice. Six out of 9 allografts survived in mice that received a single injection of rhTSG-6, whereas two out of 9 allografts survived in mice that received PBS. (b-f) The myeloperoxidase (MPO) amount and the levels of transcripts for pro-inflammatory cytokines were decreased significantly in the corneal allografts at day 7 by IV recombinant TSG-6 (35 µg) injected immediately after surgery. n=3 in each group, **(g-j)** The levels of transcripts for T cell-related cytokines were also decreased by IV recombinant TSG-6 at day 28. n=6 in each group.
**Figure 13****.** The knockdown efficiency of TSG-6 in hMSCs was approximately 82 % by real-time RT-PCR 10 hours after transfection with TSG-6-siRNA or scrambled siRNA which was the same time point when TSG-6-siRNA MSCs were injected into mice.

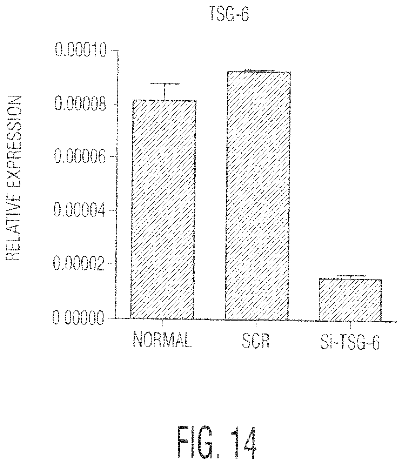

**Figure 14****. Graphic summary of the effects of hMSCs on corneal allografts.** Intravenous hMSCs suppressed inflammation in the cornea markedly early after grafting and decreased the activation and migration of dendritic cells (DCs). As a result, the immune rejection was prevented and the acceptance of corneal allografts was achieved.

The invention now will be described with respect to the following examples and comparative examples, however, the scope of the present invention is not intended to be limited thereby.

### Example 1

### Materials & Methods

### Cell preparations

Vials of frozen passage one hMSCs were obtained from the Center for the Preparation and Distribution of Adult Stem Cells (http://medicine.tamhsc.edu/irm/msc-distribution.html) that supplies standardized preparations of MSCs enriched for early progenitor cells to over 300 laboratories under the auspices of an NIH/NCRR grant (P40 RR 17447-06). All of the experiments were performed with hMSCs from one donor. The cells differentiated consistently into three lineages in culture, were negative for hematopoietic markers (CD34, CD36, CD117 and CD45), and were positive for mesenchymal markers CD29 (95%), CD44 (>93%), CD49c (99%), CD49f (>70%), CD59 (>99%), CD90 (>99%), CD105 (>99%) and CD166 (>99%). Following culture at high density for 24 hours to recover viable cells, hMSCs were plated at low density (100 cells/cm²), incubated in complete culture medium (CCM) with 16 % FBS for 8 days until approximately 70 % confluence was reached, and harvested with 0.25% trypsin/1 mM EDTA at 37 °C for 2 min. The trypsin thereafter was inactivated by adding the CCM to the cells, and the cells were washed with PBS by centrifugation at 1,200 rpm for 5 min. The cells were frozen in α-MEM with 30% FBS and 5% DMSO at a concentration of 1 x 10⁶ cells/mL. Passage two cells were used for all experiments. Following lifting the cells prior to injection, a final wash was performed using Hank's Balanced Salt Solution (HBSS; BioWhittaker, Walkersville, MD). After washing by centrifugation, the cells were suspended in HBSS at a concentration of 10,000 cells/µL for injection.

For siRNA experiments, hMSCs were transfected with siRNA for TSG-6 (sc-39819; Santa Cruz Biotechnology, Santa Cruz, CA) or scrambled siRNA (StealthTM RNAi Negative Control; Invitrogen, Carlsbad, CA) with a commercial kit (Lipofectamine RNAiMAX reagent; Invitrogen). To confirm successful knock-down of TSG-6 expression, RNA was extracted from aliquots of the cells (RNeasy Mini kit; Qiagen, Valencia, CA) and assayed for TSG-6 by real-time RT-PCR. The knockdown efficiency of TSG-6 in hMSCs was 82 to 85% from 10 to 24 hours after the start of transfection (**Figure 14**). The same cells used for assaying the knockdown efficiency were injected into mice for experiments. The cells were prepared for injection 10 hours after transfection with TSG-6-siRNA or scrambled siRNA.

### Animal model of corneal transplantation

The experimental protocols were approved by the Institutional Animal Care and Use Committee of Texas A&M Health Science Center and Seoul National University Hospital Biomedical Research Institute. Eight-week-old female B6 mice (C57BL/6J, H-2b, Charles River Laboratories International, Inc. Wilmington, MA) were used as corneal donors and BALB/c mice (BALB/cAnNCr1, H-2d, Charles River Laboratories International, Inc.) served as corneal transplant recipients.

Central 2-mm diameter corneal grafts were excised from donor corneas of B6 mice using a 2.0 mm trephine (Katena Products, Inc., Denville, NJ). The recipient corneal graft bed was prepared by removing a central 1.5-mm diameter button in recipient corneas of BALB/c host mice with a 1.5 mm trephine (Katena Products, Inc.). The prepared donor corneal grafts were placed in the recipient bed and secured with eight interrupted 11-0 nylon sutures. Syngeneic autografts (BALB/c-to-BALB/c) served as experimental controls and were performed in the same fashion. The lids were closed with an 8-0 nylon tarsorrhaphy which was maintained (except for clinical evaluation) until graft rejection developed. All grafts were evaluated three times weekly for 6 weeks. Graft rejection was defined as a complete loss of graft transparency (i.e., the pupil margin and iris structure are not visible through the graft). Recipient mice received 1 x 10⁶ hMSCs in 100 µL HBSS via tail vein either once (immediately after surgery) or twice (one day before surgery and immediately after surgery). HBSS was injected IV in the control group. For IV TSG-6 experiments, each mouse received 35 µg of recombinant human (rh)TSG-6 (R&D Systems, Minneapolis, MN) in 100 µL PBS or PBS 100 µL via tail vein

### Histopathology

For tissue extraction, following sacrifice of the mice, the cornea was excised and fixed in 10% paraformaldehyde. The cornea was cut into 4 µm sections and stained with H&E or subjected to immunohistochemistry. The formalin-fixed corneal sections were deparaffinized with ethanol and antigen was retrieved using a steamer in epitope retrieval solution (IHC WORLD, Woodstock, MD). Primary antibodies used were as follows: rabbit polyclonal antibody to CD3 (ab5690, Abcam, Cambridge, MA), rat monoclonal antibody to F4/80 (ab6640, Abcam), rabbit polyclonal antibody to iNOS (ab15323, Abcam), mouse monoclonal antibody to MRC (ab8918, Abcam), and mouse monoclonal antibody to MHC class II I Ad (ab64531, Abcam). A DAPI solution (VECTASHIELD Mounting Medium; Burlingame, CA) was used for counterstaining.

### Real-time RT-PCR assays of cornea and lungs

For RNA extraction, the cornea or lung was minced into small pieces, lysed in RNA isolation reagent (RNA Bee, Tel-Test Inc., Friendswood, TX), and homogenized using a motor-driven homogenizer. Total RNA was then extracted using RNeasy Mini kit (Qiagen) and used to synthesize double-stranded cDNA by reverse transcription (SuperScript III, Invitrogen). Real-time amplification was performed using TaqMan Universal PCR Master Mix (Applied Biosystems, Carlsbad, CA). An 18s rRNA probe (TaqMan Gene Expression Assays ID, Hs03003631_g1) was used for normalization of gene expression. For all the PCR probe sets, TaqMan Gene Expression Assay kits were purchased from Applied Biosystems. The assays were performed in triple technical replicates for each biological sample.

### Real-Time RT-PCR standard curve for hGAPDH

A standard curve was generated by adding serial dilutions of hMSCs to mouse tissue as previously described. (Roddy, 2011; Lee, 2009). Briefly, 10-100,000 hMSCs were added to a mouse cornea. Following RNA extraction (RNeasy Mini kit; Qiagen), cDNA was generated by reverse transcription (SuperScript III; Invitrogen) using 1 µg total RNA. A human-specific GAPDH (hGAPDH) primer and probe set (TaqMan Gene Expression Assays ID, GAPDH HS99999905_05) was used. The values were normalized to total eukaryotic 18s rRNA. The standard curve was made based on hGAPDH expression from a known number of hMSCs added to one mouse cornea.

### ELISAs

For protein extraction, the cornea was minced into small pieces and lysed in tissue extraction reagent (Invitrogen) containing protease inhibitor cocktail (Roche, Indianapolis, IN). The samples were sonicated on ice (Ultrasonic Processor, Cole Parmer Instruments, Vernon Hills IL). After centrifugation at 12,000 rpm at 4°C for 20 min, the supernatant was collected and assayed by ELISA for IL-1β, IFN-γ, and IL-12p70 (Mouse Duoset kit; R&D Systems, Minneapolis, MN), CCR7 (USCN Life Science, Inc., Missouri City, TX), and MPO (HyCult biotech, Plymouth Meeting, PA).

### Flow cytometry

DLNs were harvested from transplanted animals at days 7 and 28 after surgery. Each sample from an individual animal was prepared and anlayzed separately. No pooling of lymph node cells between animals was done. DLNs were placed and minced between the frosted ends of two glass slides in RPMI media containing 10% FBS and 1% penicillin-streptomycin. Cell suspensions were collected, and incubated for 30 min at 4 °C with tluorescein-conjugated antimouse antibodies. The primary antibodies used were as follows: I-A^{d}-PE, CD11b-FITC, CD11c-allophycocyanin, CD3-FITC, CD4-PE, and CD8- allophycocyanin (eBioscience, San Diego, CA). Three color phenotypic analyses were performed using a FACSCan to flow cytometer (BD BioSciences, Mountain View, CA). A total of 20,000 events from each sample were collected. The gate was set on either I-A^{d+} or CD3⁺ cell population, and further analysis of surface markers was done within this gate. Data were analyzed using Flowjo program (Tree Star, Inc., Ashland, OR).

### Statistical analysis

Survival analysis was performed using SPSS software (SPSS 12.0, Chicago, IL). The Kaplan-Meier method was used to evaluate the overall cumulative probability of graft survival, and the life-table method was used to estimate the median time to graft rejection. The graft survival between the groups was compared using the Breslow (Generalized Wilcoxon) test. Comparisons of parameters other than graft survival were made among the groups using one-way ANOVA using SPSS software. Differences were considered significant at *p* < 0.05.

### Results

### IV hMSCs prolonged the survival of corneal allografts

To determine whether IV hMSCs prolong the survival of corneal allografts, we performed orthotropic corneal allotransplantation using C57BL/6 mice (H-2^{b}) as donors and BALB/c (H-2^{d}) as recipients. Recipient mice received 1 x 10⁶ hMSCs IV either once immediately after surgery (day 0) or twice at one day before surgery (day -1) and again immediately after surgery (day 0). Hank's Balanced Salt Solution (HBSS) was injected IV as a vehicle control. Syngeneic corneal autografts (BALB/c-to-BALB/c) were performed to serve as negative controls. For the follow-up period of 42 days, 7 of 12 B6 corneal grafts in BALB/c mice (allografts) were rejected within 28 days with a mean survival time of 21.3 days, while all of the BALB/c corneal grafts in BALB/c mice (autografts, n=12) survived (**Fig**. **1A**). IV hMSCs prolonged the survival of corneal allografts significantly. Eleven out of 12 allografts remained free of rejection in mice that received two injections of IV hMSCs (p=0.006, vs. allografts in the HBSS group), and 9 of 12 allografts survived in mice that received a single injection of hMSCs (p=0.047, vs. allografts in the HBSS group).

We performed histological studies on the grafts at day 28. In the rejected allografts of HBSS-injected animals, hematoxylin-eosin (H&E) staining of sections showed extensive infiltration of inflammatory cells (**Fig**. **1B**). In contrast, inflammatory infiltrates were markedly decreased in the allografts from mice treated with hMSCs. Similar results were observed by immunostaining of the sections for CD3⁺ T cells (**Fig**. **1C**). There was extensive infiltration of CD3⁺ T cells in the rejected grafts from vehicle control animals and minimal infiltration of CD3⁺ T cells in the allografts from mice that received hMSCs.

Therefore, the data demonstrated that peri-transplant injection of IV hMSCs prolonged the survival of corneal allografts and prevented rejection. Two injections (day -1 and day 0) were more effective than a single injection (day 0).

### IV hMSCs suppressed early inflammation and late rejection of corneal allografts

In order to examine the effects of hMSCs, we analyzed corneal grafts for the time course of expression of inflammation- and immune-related molecules. Because the rejection of corneal allografts occurred by day 28 and the surviving grafts remained free of rejection after day 28 (**Fig**. **1A**), we analyzed corneal grafts at day 28 for the immune rejection, and the grafts at days 3 and 7 for the early surgery-induced inflammation. We found that both autografts and allografts demonstrated the same early increases at days 3 and 7 of the inflammatory cytokines IL-6, IL-1β, and IL-12 as well as myeloperoxidase as a semi-quantitative measure of neutrophil infiltration (**Figs 2A, 2B** **and** **Fig. 3**). (Oh, et al., Proc. Nat. Acad. Sci., Vol. 107, pgs. 16875-16880 (2010). This finding indicates that the inflammation was induced by surgery and the similar amount of damage was applied to tissues by surgery between auto- and allografts. In contrast, there were marked differences between autografts and allografts in the late immune response. In allografts but not in autografts, there was a gradual increase up to day 28 in the transcript levels of T cell-derived cytokines (IL-2 and IFN-γ) and effector molecules implicated in the allograft rejection (granzyme A, granzyme B, and perforin) (**Figs 2A, 2B** **and** **Fig. 4**). (Choy, et al., Cell Death Differ., Vol. 17, pgs. 567-576 (2010)). IV infusion of hMSCs decreased both early inflammatory phase and late immune response (**Fig. 2C**). At days 3 and 7, the levels of inflammatory cytokines were reduced markedly in allografts from mice that received hMSCs. The levels of the transcripts for IL-6 and IL-1β were reduced by about half, and the transcript for IL-12a was reduced to baseline levels. Similar decreases were seen at day 7 in levels of myeloperoxidase and ELISA for IL-1β and IL-12 (**Fig. 3**). The decrease in the inflammatory phase produced by hMSCs was accompanied by a decrease in the immune response. At 28 days, there was a marked decrease in the levels of transcripts for IL-2, IFN-γ, granzyme A, granzyme B, and perforin in the allografts from mice that received hMSCs as well as the protein level of IFN-γ (**Fig. 2C** **and** **Fig. 4**). Also, flow cytometry demonstrated that the number of CD44⁺ CD69⁺ cells as markers for activated CD4 T cells was decreased in DLNs in mice treated with hMSCs (Figure 5).

Therefore, the results indicated that hMSCs suppressed the surgery-induced inflammation in the early postoperative period and, apparently as a result, decreased the subsequent immune rejection of corneal allografts.

### IV hMSCs decreased activation of antigen presenting cells

Previous studies demonstrated that the principal antigen-presenting cells (APCs) of the cornea. Langerhans cells (LCs) reside in basal epithelium in the limbal area of the cornea. (Forrester, et al., Immunol. Rev., Vol. 234, pgs. 282-304 (2010)). Also, CD11b⁺ CD11c⁺ cells having dendritic morphology are present in the anterior stroma, and a population of CD11b⁺ CD11c⁻ cells having macrophage morphology is present in the posterior stroma. (Hamrah, et al., J. Leukocyte Biol., Vol. 74, pgs. 172-178 (2003)). In response to inflammatory insults including transplantation surgery, APCs undergo maturation by overexpressing major histocompatibility complex (MHC) class II. (Dana, Invest. Ophthalmol. Vis. Sci., Vol. 45, pgs. 722-727 (2004); Dana, Trans. Am. Ophthalmol. Soc., Vol. 105, pgs. 330-343 (2007)). Activated APCs, most of which are of host origin, take up graft-derived antigens in the cornea and migrate to DLNs, where they present antigens to host T cells causing the T cell-mediated immune rejection. (Forrester, 2007; Kuffova, et al., J. Immunol., Vol. 180, pgs. 1353-1361 (2008)). Thus, we next examined whether the decrease in inflammation by IV hMSCs might lead to a reduction in the activation of APCs in the cornea and DLNs. First, we examined the whole-mounted epithelial sheets of the cornea for host-derived MHC class II (murine Ia^{d}) at one week after transplantation. We selected the one-week time-point, because it is the time at which allosensitization takes place and allorejection is not yet initiated. (Forrester, 2007). Therefore, it allowed us to examine the afferent sensitization arm of alloimmunity. Immunostaining showed that the number of MHC class II⁺ cells in the cornea was markedly lower in the allografts from mice treated with hMSCs, compared to the autografts or allografts without hMSCs (**Fig. 6**). Next, we analyzed subsets of MHC class II⁺ cell population in DLNs (cervical LNs ipsilateral to the transplanted eye). Flow cytometry showed that the proportions of DCs, both MHC II⁺ CD11b⁺ CD11c⁺ cells and MHC II⁺CD11b⁻CD11c⁺ cells, were decreased significantly in mice treated with hMSCs (**Fig. 7**). Two injections of hMSCs (day -1 and day 0) were more effective than a single injection (day 0). In addition to DCs, the proportion of MHC II⁺ CD11b⁻ CD11c⁺ cells representing macrophages was decreased significantly in the group treated with IV hMSCs. The findings of similar increases in APCs in the cornea and DLNs between auto- and allografts suggested that a transplant procedure and surgically-induced inflammation contributed to activation of APCs, which is consistent with the data reported previously. (Dana, 2007). Treatment with IV hMSCs was accompanied by reduced activation of APCs in the cornea and DLNs as well as reduced inflammation in the grafts. The data, therefore, indicated that the afferent limb of the alloimmune response was inhibited by hMSCs.

### IV administered hMSCs did not engraft in the corneal allografts.

Previous reports showed that the vast majority of hMSCs infused IV in mice were trapped in lungs and disappeared with a half-life of about 24 hours without long-term engraftment into injured tissues such as the cornea or heart. (Roddy, et al., Stem Cells, Vol. 29, pgs. 1572-1579 (2011); Lee, et al., Cell Stem Cell, Vol. 5, pgs. 54-63 (2009)). To determine whether hMSCs engrafted in the transplanted cornea after IV injection, we carried out quantitative RT-PCR assays for human-specific GAPDH in the corneas from mice that received two IV infusions of 1 x 10⁶ hMSCs at day -1 and day 0 of corneal transplantation (Figure 8 and Table 1). The results shown in Figure 8 and Table 1 demonstrated that less than 10 hMSCs were present in corneas 10 hours to 28 days after the transplants. Therefore, the beneficial effects of the hMSCs were not explained by the IV administered cells engrafting in the cornea.

**Table 1**

| The standard curve for the number of human mesenchymal stem cells (hMSCs) in one mouse cornea based on the expression of human-specific GAPDH (hGAPDH) relative to total eukaryotic 18s rRNA. The expression levels of hGAPDH in the rat cornea at day 1 and day 3 after intravenous (IV) injection of hMSCs (1 x 10⁶ cells per animal). | | | | | |
|---|---|---|---|---|---|
| No. of hMSCs added per one mouse cornea | Value of ΔCT (cycling time of hGAPDH - cycling time of 18s rRNA) | | | | |
| | Average | | | Standard deviation | |
| 100,000 | -2.001371 | | | 0.017618 | |
| 10,000 | | 0.616121 | | 0.054784 | |
| 1,000 | | 5.360307 | | 0.397577 | |
| 100 | | 7.957465 | | 0.440978 | |
| 10 | | 12.07501 | | 0.345224 | |
| | | | | | |

| Animal No. (injected with 1x10⁶ hMSCs per animal) | Value of ΔCT (cycling time of hGAPDH - cycling time of 18s rRNA) | | | | |
|---|---|---|---|---|---|
| | 10 hrs | 1 day | 3 days | 7 days | 28 days |
| 1 | 16.84249 | 20.90485 | Not amplified | Not amplified | Not amplified |
| 2 | 19.03035 | 21.57985 | Not amplified | Not amplified | Not amplified |
| 3 | 18.60903 | 21.22632 | Not amplified | Not amplified | Not amplified |
| 4 | 18.24448 | 20.59102 | Not amplified | Not amplified | Not amplified |
| 5 | 17.10939 | 21.82082 | Not amplified | Not amplified | Not amplified |

### IV hMSCs trapped in lungs were activated to express the anti-inflammatory gene/protein TSG-6

Because the majority of IV hMSCs were trapped in lungs (Lee, 2009) and did not engraft in the cornea (Figure 8 and Table 1), we examined the hypothesis that the effects of hMSCs on corneal grafts were mediated by trophic factors produced from the cells trapped in lungs. We used human-specific quantitative RT-PCR assays to screen the lungs 10 hours after corneal allotransplantation in mice that received IV hMSCs twice at day -1 and day 0. Data revealed that about 10% of injected cells were present in lungs (**Figure 9**). We assayed for the expression of immunomodulatory and anti-inflammatory molecules that have been shown previously to be secreted by MSCs: COX2, NOS2, IDO, CCL2, TGF-β, TSG-6, STC-1, and PTX3. (English, et al., Cell Stem Cell, Vol. 7, pgs. 431-442 (2010); Lee, et al., J. Cell. Biochem., Vol. 112, pgs. 3073-3078 (2011)). We found that the most highly up-regulated human transcript was for the anti-inflammatory protein TSG-6 (113.8-fold) (**Figure 10**).

### IV hMSCs with TSG-6 siRNA knockdown did not either reduce the early inflammation or prolong allograft survival.

To explore the role of TSG-6 in preventing graft rejection, we knocked down the expression of TSG-6 in hMSCs by transient transfection with siRNA and injected the cells into mice with corneal allografts at day -1 and day 0 of surgery. Three out of 6 allografts were rejected in mice that received a two-time injection of hMSCs with TSG-6 knockdown, whereas all (6/6) of the allografts remained free of rejection at 28 days in mice that received a two-time injection of hMSCs with scrambled siRNA control (p=0.047; Generalized Wilcoxon test) (**Fig. 11A**). Additionally, hMSCs with TSG-6 knockdown were not effective in suppressing corneal inflammation at day 7 (**Fig. 11B**). Also, levels of transcripts for activated T cell-derived cytokines and effector enzymes in corneal grafts were not suppressed by hMSCs with TSG-6 knockdown, whereas hMSCs with scrambled siRNA significantly decreased the levels of transcripts for T-cell cytokines and enzymes (**Figs 11C-H**).

### IV injection of recombinant TSG-6 reduced early inflammation of the allografts and late rejection of corneal allografts

Next, we tested the hypothesis that systemically-administered rhTSG-6 could reproduce the effects of IV hMSCs by reducing the surgery-induced inflammation in corneal allografts. We administered 35 µg of rhTSG-6 in 100 µl PBS by tail vein injection immediately after corneal allotransplantation. The survival of corneal allografts was prolonged significantly in mice treated with rhTSG-6, compared to PBS-injected controls (the mean survival time: 25.6+1.5 days in the TSG-6 treated grafts and 18.6± 3.1 days in the PBS-treated grafts; p=0.042; Generalized Wilcoxon test) (**Fig. 12**). The expression of MPO and pro-inflammatory cytokines (IL-6, IL-1β, IL-12a, and IL-12b) in the cornea at day 7 was decreased significantly following rhTSG-6 injection (**Fig. 12B-F**). Also, levels of transcripts for T cell-related cytokines, IL-2, IFN-γ, granzyme B, and perforin, were decreased significantly in the allografts from mice treated with TSG-6 at day 28 (**Fig**. **12G-J**).

### Discussion

As summarized in **Figure 13****,** the results demonstrated that IV hMSCs increased the survival of the allografts without long-term engraftment by aborting the early inflammatory response and by decreasing activation of APCs in corneas after transplantation surgery.

Our data are consistent with the current paradigm that hMSCs can produce therapeutic benefits without engraftment into injured tissues and primarily by up-regulating the genes that modulate excessive inflammatory and immune reactions.(Prockop, et al., J. Cell. Mol. Med., Vol. 14, pgs. 2190-2199 (2010); Uccelli, et al., Curr. Opin. Immunol., Vol. 22, pgs. 768-774 (2010). In the present experiment, the multi-functional anti-inflammatory protein TSG-6 (Milner, et al., Biochem. Soc. Trans., Vol. 34, pgs. 446-450 (2006); Wisniewski, et al., Cytokine Growth Factor, Rev., Vol. 15, pgs. 129-146 (2004) accounted for beneficial effects of the hMSCs that were infused IV. Since pro-inflammatory cytokines, including IL-1, play a critical role in recruitment, activation, and migration of APCs such as LCs (Dana, 2004; Dana, 2007), suppression of inflammation early after grafting by hMSCs might contribute to a prolonged survival of corneal allografts through inhibition of the afferent loop of the immune response.

In addition to suppressing inflammation, MSCs also may have other effects on the immune system. A large number of *in vitro* and *in vivo* data demonstrate that MSCs can be immunosuppressive through their interaction with a broad range of immune cells (T and B cells, regulatory T cells, NK cells, DCs, macrophages, and neutrophils) and by secreting a number of molecules such as IDO, PGE₂, nitric oxide. CCL2, TGF-β, TSG-6, IL-10, or HLA-G. (Uccelli, 2010; Siegel, et al., Transplantation, Vol. 87, pgs. 545-549 (2009); Ren, et al., Cell Stem Cell, Vol. 2, pgs. 141-150 (2008); Rafei, et al., J. Immunol., Vol. 182, pgs. 5994-6002 (2009); Crop, et al.. Transpl. Int., Vol. 22, pgs. 365-376 (2009)). This considerable diversity and discrepancy in experimental findings for the immune-modulatory mechanisms of MSCs probably reflects their remarkable ability to respond to the microenvironments of injured tissues. In the present experiment, we injected hMSCs IV one day prior to and at the time of transplantation. Although the cells rapidly disappeared from the system after injection (Lee, 2009), the MSCs decreased the early surgery-induced inflammation significantly by up-regulating the anti-inflammatory molecule TSG-6. Notably, in our present experimental setting, the expression of immunomodulatory and anti-inflammatory molecules that have been shown previously to be secreted by MSCs (COX2, NOS2, IDO, CCL2, TGF-β, STC-1, and PTX3) was not up-regulated in hMSCs at the transcriptional level. We cannot rule out, however, the possibility that consistent expression of molecules other than TSG-6 at protein levels might contribute to the action of hMSCs observed in the current study. Similar results were obtained with IV recombinant TSG-6. Also, administration of hMSCs may have pre-conditioned the complex systems for inflammatory and immune responses so that the effects were apparent after most of hMSCs no longer were detected.

One of the critical observations here was that the hMSCs were more effective if they were infused IV both the day before surgery and immediately after the surgery than if they were infused only immediately after the surgery. The result may reflect the fact that hMSCs do not express therapeutic proteins such as TSG-6 in culture unless activated by pro-inflammatory cytokines such as TNF-α or IFN-γ, and they are not activated to express TSG-6 until about 10 hours after they are trapped in the lungs following IV infusion. (Lee, 2009). Therefore, the preoperative administration of hMSCs might be more effective to reduce the surgery-induced inflammation as shown in this study.

We used a mouse model in the present study because corneal allotransplantation is the most well-studied in mice and thus the temporal sequence of events from the introduction of the alloantigen to immune rejection is well-established in this model. (Forrester, 2010; Catron, J. Exp. Med., Vol. 203, pgs. 1045-1054 (2006); Kuffova, et al., Transplantation, Vol. 72, pgs. 1292-1298 (2001)). The surgical procedure of transplanting corneas in mouse eyes, however, might have induced more severe tissue damage and surgically-induced inflammation compared to transplantation in humans. Further studies in larger animal models will be beneficial.

The results may explain well the beneficial effects of MSCs observed in various animal models of organ transplantation such as heart, lung, skin, and islets. (Crop, 2009; Casiraghi, et al., J. Immunol., Vol. 181. pgs. 3933-3946 (2008); Jawinen, et al., J. Immunol., Vol. 181, pgs. 4389-4396 (2008); Sbano, et al., Arch. Dermatol. Res., Vol. 300, pgs. 115-124 (2008); Ding, et al., Transplantation, Vol. 89, pgs. 270-273 (2010)). In addition, the data may extend upon previous studies that focused mostly on examining immune tolerance as a mechanism for MSCs to improve transplant survival. Early inflammatory responses also may be inviting targets for therapy. The results presented here add another rationale for using MSCs as a primary or secondary therapy to decrease the need for pharmacological immunosuppression in patients with transplants. Moreover, since IV administration of recombinant TSG-6 reproduced many of the beneficial effects of the hMSCs, the results raise the possibility that therapy with the protein may be more practical than therapy with the cells.

In summary, our data provide a basis for using either MSCs or TSG-6 to improve the survival of transplants of the cornea and possibly other organs.

It is to be understood, however, that the scope of the present invention is not to be limited to the specific embodiments described above. The invention may be practiced other than as particularly described and still be within the scope of the accompanying claims.

### References

1. Hoogduijn MJ, et al. Advancement of mesenchymal stem cell therapy in solid organ transplantation (MISOT). Transplantation. 2010;90:124-6.
2. Developmental Committee of the European Group for Blood and Marrow Transplantation. Mesenchymal stem cells for treatment of steroid-resistant, severe, acute graft-versus-host disease: a phase II study. Lancet. 2008;371:1579-86.
3. English K, French A, Wood KJ. Mesenchymal stromal cells: facilitators of successful transplantation? Cell Stem Cell. 2010;7:431-42.
4. Popp FC, Eggenhofer E, Renner P, et al. Mesenchymal stem cells can affect solid organ allograft survival. Transplantation. 2009;87:S57-62.
5. Singer NG, Caplan AI. Mesenchymal stem cells: mechanisms of inflammation. Annu Rev Pathol. 2011;6:457-78.
6. Uccelli A, Moretta L, Pistoia V. Mesenchymal stem cells in health and disease. Nat Rev Immunol. 2008;8:726-36.
7. Forrester JV, Xu H, Kuffová L, Dick AD, McMenamin PG. Dendritic cell physiology and function in the eye. Immunol Rev. 2010 Mar:234:282-304.
8. Catron DM, Rusch LK, Hataye J, Itano AA, Jenkins MK. CD4+ T cells that enter the draining lymph nodes after antigen injection participate in the primary response and become central-memory cells. J Exp Med. 2006;203:1045-1054.
9. Kuffova L, et al. Kinetics of leukocyte and myeloid cell traffic in the murine corneal allograft response. Transplantation. 2001;72:1292-1298.

## Claims

1. A composition comprising TSG-6 protein, or biologically active fragment thereof for use in a method of preventing or treating rejection of a corneal transplant in an animal.

2. The composition of Claim 1 wherein said composition further comprises mesenchymal stem cells.

3. The composition of Claim 1 where said composition further comprises at least one immunoregulator and/or at least one immunosuppressive agent.

## Patentansprüche

1. Zusammensetzung, die TSG-6-Protein oder ein biologisch aktives Fragment davon umfasst, zur Verwendung in einem Verfahren zur Verhinderung oder Behandlung einer Abstoßung eines Hornhauttransplantats bei einem Tier.

2. Zusammensetzung nach Anspruch 1, wobei die Zusammensetzung ferner mesenchymale Stammzellen umfasst.

3. Zusammensetzung nach Anspruch 1, wobei die Zusammensetzung ferner mindestens einen Immunregulator und/oder mindestens ein immunsuppresives Mittel umfasst.

## Revendications

1. Composition comprenant une protéine TSG-6 ou un fragment biologiquement actif de celle-ci pour une utilisation dans un procédé de prévention ou de traitement d'un rejet de greffe de cornée chez un animal.

2. Composition selon la revendication 1 , où ladite composition comprend en outre des cellules souches mésenchymateuses.

3. Composition selon la revendication 1 , où ladite composition comprend en outre au moins un immunorégulateur et/ou au moins un agent immunosuppresseur.


### SEQUENCE LISTING

<110> The Texas A&M University System Scott & White Healthcare Prockop, Darwin J. Oh, Joo Youn Lee, Ryang Hwa Yu, Ji Min
<120> Prevention and Treatment of Transplant Rejection with Mesenchymal Stem Cells and/or TSG-6 Protein
<130> 680251-18
<140> PCT/US2012/062972
<141> 2012-11-01
<150> US 61/555,717
<151> 2012-11-04
<160> 1
<210> 1
   <211> 277
   <212> PRT
   <213> Homo sapiens
<220>
   <221> TSG-6 protein
<400> 1
   Met Ile Ile Leu Ile Tyr Leu Phe Leu Leu
                       5                   10
   Leu Trp Glu Asp Thr Gln Gly Tyr Gly Phe
                       15                  20
   Lys Asp Gly Ile Phe His Asn Ser Ile Trp
                       25                  30
   Leu Glu Arg Ala Ala Gly Val Tyr His Arg
                       35                  40
   Glu Ala Arg Ser Gly Lys Tyr Lys Leu Thr
                       45                  50
   Tyr Ala Glu Ala Lys Ala Val Cys Glu Phe
   55 60
   Glu Gly Gly His Leu Ala Thr Tyr Lys Gln
   65 70
   Leu Glu Ala Ala Arg Lys Ile Gly Phe His
   75 80
   Val Cys Ala Ala Gly Trp Met Ala Lys Gly
   85 90
   Arg Val Gly Tyr Pro Ile Val Lys Pro Gly
   95 100
   Pro Asn Cys Gly Phe Gly Lys Thr Gly Ile
   105 110
   Ile Asp Tyr Gly Ile Arg Leu Asn Arg Ser
   115 120
   Glu Arg Trp Asp Ala Tyr Cys Tyr Asn Pro
   125 130
   His Ala Lys Glu Cys Gly Gly Val Phe Thr
   135 140
   Asp Pro Lys Gln Ile Phe Lys Ser Pro Gly
   145 150
   Phe Pro Asn Glu Tyr Glu Asp Asn Gln Ile
   155 160
   Cys Tyr Trp His Ile Arg Leu Lys Tyr Gly
   165 170
   Gln Arg Ile His Leu Ser Phe Leu Asp Phe
   175 180
   Asp Leu Glu Asp Asp Pro Gly Cys Leu Ala
   185 190
   Asp Tyr Val Glu Ile Tyr Asp Ser Tyr Asp
   195 200
   Asp Val His Gly Phe Val Gly Arg Tyr Cys
   205 210
   Gly Asp Glu Leu Pro Asp Asp Ile Ile Ser
   215 220
   Thr Gly Asn Val Met Thr Leu Lys Phe Leu
   225 230
   Ser Asp Ala Ser Val Thr Ala Gly Gly Phe
   235 240
   Gln Ile Lys Tyr Val Ala Met Asp Pro Val
   245 250
   Ser Lys Ser Ser Gln Gly Lys Asn Thr Ser
   255 260
   Thr Thr Ser Thr Gly Asn Lys Asn Phe Leu
   265 270
   Ala Gly Arg Phe Ser His Leu
   275